# EUROPEAN PATENT SPECIFICATION

(11) **EP 0 998 853 B1**
(45) Date of publication and mention of the grant of the patent: **10.09.2003**
(21) Application number: 99660174.6
(22) Date of filing: 03.11.1999
(51) Int. Cl.: A23K 1/16, A23K 1/18, C07C 57/12

(54) **Feed and method for its preparation**
Futter und Verfahren zur Herstellung
Aliment pour animaux et procédé de préparation

(30) Priority: 06.11.1998 FI 982412
(43) Date of publication of application: 10.05.2000
(73) Proprietor: Rehuraisio OY, 21200 Raisio (FI)
(72) Inventor: Holma, Merja, 21200 Raisio (FI)
(74) Representative: Grew, Eva Regina

(56) References cited:
- WO-A-99/20123
- GB-A- 2 113 521
- US-A- 4 169 843
- US-A- 4 642 317
- US-A- 5 770 247
- SELNER, D. R., AND SCHULTZ, L. H.: "Effect of feeding oleic or hydrogenated vegetable oils to lactating cows." JOURNAL OF DAIRY SCIENCE, vol. 63, 1980, pages 1235-1241, XP002131007
- ERDMAN, R. A., AND TETER, B. B.: "The role of trans fatty acids in diet induced milk fat depression in dairy cows" JOURNAL OF DAIRY SCIENCE, vol. 78, no. Suppl. 1, 1995, page 229 XP002131008
- SCHNEDIER ET AL.: "Feeding Calcium Salts of Fatty Acids to Lactating Cows" JOURNAL OF DAIRY SCIENCE, vol. 71, 1988, pages 2143-2150, XP002131009

## Description

An object of this invention is a feed, such as a compound or complete feed, intended specifically for ruminants, such as for cows, which feed contains conventional, advantageously low-fat grain-based feed components, as well as conventional feed additives and other adjuvants, and by means of which feed the composition of milk fat can be changed in a healthier direction to satisfy the expectations of the consumers.

An object of the invention is also a method for the preparation of said feed, and a fatty acid mixture usable as an additive in feeds, as well as a method for the preparation and the use of the said fatty acid mixture.

The consumption of milk fat has decreased among Finns by almost 25% during the last 20 years. According to nutritional recommendations the overall goal is a decrease by 45% in the consumption of milk fat as compared to the 1975 year level. The average consumption of milk fat was then 55 g per person a day. According to present recommendations the daily consumption of milk fat should decrease to below 30 g.

The reason for the strong decrease in milk fat comsumption and for the attempts at further decreasing its consumption rests firstly in the milk fatty acid composition, which increases the risk of cardiovascular diseases and increases the cholesterol level in the blood. On the other hand, there are efforts to decrease total fat consumption in the human diet, because obesity has become a new health risk in the western countries.

The biggest problem in milk fat is the high level of middle length saturated fatty acids (lauric (12:0), myristic (C14:0) and palmitic acid (C16:0)) which have been shown to increase the risk of cardiovascular disease. On the other hand, milk fat is poor in oleic acid (C18:1 cis) as well as in polyunsaturated fatty acids. The newest focus of interest is the conjugated linoleic acid, that is CLA, contained in milk fat, which has been shown to decrease the risk of cancer in many animal and *in vitro* tests. Also the trans-vaccenic acid (C18:1 trans 11) contained in milk fat is a desired component, because it has been shown to convert to CLA in the tissues under the effect of the delta-9-desaturase enzym.

A typical composition for milk fat today and the aim as regards changing the composition with respect to certain fatty acids is the following:

| | Present | Desired |
|---|---|---|
| C12:0, C14:0 and C16:0 | 50 | 30 |
| C18:1 (oleic acid) | 20 | 40 |
| CLA | 0.5 | 1.5 |
| C18:1 Mans 11 | 1 | 4. |

It is possible to affect the composition of the milk and the quality of the fat to some degree by means of the diet provided for the cows. It is known that the administration of oils to cows makes the milk fat softer. The oil diet has, however, negative effects both on the rumen function and the milk quality. By protecting the fatty acids of plant oils it is possible to decrease the negative effects on the rumen, but no vaccenic acid nor CLA is then obtained in the milk fat.

When a cow eats unprotected fat with its feed, the fat is exposed to the action of the rumen microbes. The lipase enzymes produced by the microbes hydrolyse ester bonds in the triglycerides, whereby glycerol and free fatty acids are formed. The hydrolysis is rapid, because already after an hour after feeding, a major part of the triglycerides is hydrolysed, leading to an increase in the free fatty acids in the rumen. The fatty acids have to be in free form before the the microbes can use them. After liberation, the polyunsaturated fatty acids start to hydrogenate in the rumen. The biohydrogenation progresses stepwise so that the end product is stearic acid. The biohydrogenation process is not complete, but many different fatty acids are formed in the rumen as hydrogenation intermediates. Especially trans-vaccenic acid is a limiting step in the hydrogenation, whereby the end product is often a mixture of this isomer and stearic acid.

Isomeric transformations often occur in the intermediates and possible end products of hydrogenation. Isomeric transformations of fatty acids are often associated with a change of location of a double bond and changes in the chain length. Conjugated linoleic acid, that is CLA, is one intermediate in hydrogenation, some of which flows from the rumen to the lower gastrointestinal tract. Of the CLA of the milk fat, some originates from the rumen, some is formed in the mammary gland tissue in the desaturation of vaccenic acid.

Biohydrogenation protects the rumen microbes, because polyunsaturated fatty acids are toxic especially to cellulolytic bacteria. The side effects of fats can be reduced by preventing the hydrolysis of the fats. The hydrolysis of the fats can be reduced, for example, by protecting the fats with formaldehyde treated casein. Another alternative is to form insoluble salts of the fatty acids, especially calcium salts, in which case rumen hydrogenation can be avoided.

By protecting the triglycerides or the fatty acids against rumen microbes it is possible to increase the level of polyunsaturated fatty acids in the milk, but not of CLA nor trans-vaccenic acid. A high level of polyunsaturated protected fatty acids in the milk gives rise to taste flaws and preservation problems, wherefore their content cannot be very high.

In the US-patent 5,770,247 a method is disclosed for increasing the level of CLA in milk by feeding cows unprotected plant oil which contains over 30% linoleic and over 30% linolenic acid. By infusing partly hydrogenated plant oil while bypassing the rumen the CLA level of the milk has also increased. Simultaneously the fat content of the milk has decreased in some tests. Compositions for supplying fatty acids to ruminants comprising feeding the fatty acids to the ruminants in the form of their calcium salts in order to prevent problems with disturbance of the rumen microorganism population are known from US-A-4 642 317. Hydrogenated plant oil contains trans-fatty acids, which are known to inhibit the fat synthesis of the mammary gland, cf. for example US 5,416,115. The milk protein content, however, has not decreased as a result of giving partly hydrogenated oil, as is the case generally when feeding polyunsaturated oils. Trans-vaccenic acid included in partly hydrogenated plant oil in turn increases the CLA content of the milk. A typical industrially hydrogenated plant oil (soybean oil) contains 30-35% trans-fatty acids. Hydrogenated plant oil cannot, however, be fed as such because a major part of its fatty acids become a target for the rumen biohydrogenation.

According to the invention a feed mixture has now been developed, by means of which the fatty acid composition of milk can be changed to be closer to the goal, that is a fatty acid composition is obtained in the milk wherein the proportion of saturated fatty acids of middle chain length is decreased and simultaneously the proportion of oleic acid, CLA and trans-vaccenic acid has increased. The feed mixture according to the invention does not, however, disturb rumen functions. The milk protein level remains unchanged and the fat content decreases somewhat, however, the milk production increases.

According to the invention this objective is reached by means of a feed, which contains a protected trans-fatty acid mixture. The object of the invention is thus a feed which contains a mixture of fatty acids, wherein the proportion of mono- and polyunsaturated C18-trans-fatty acids which are protected against biohydrogenation is over 50 % by weight.

From the point of view of the invention it is advantageous and preferred that the total fat in the diet is small. Thus preferably low-fat grain-based raw materials are used as feed components. Barley (fat approximately 2.2 % by weight of the dry matter), oat (fat approximately 5 % by weight of the dry matter) and wheat bran (fat approximately 3-4 % by weight of the dry matter) can be given as examples of such raw materials. The proportion of glycerol in the fat naturally varies somewhat depending on the fat, but is typically approximately 10 % by weight, the remaining part being fatty acids. From the point of view of the invention it is essential that the proportion of trans-fatty acids in the feed has been substantially increased by adding thereto a mixture of fatty acids which has been concentrated with respect to the trans-fatty acids.

Consequently the objective of the invention is reached by adding to a feed mixture a fatty acid mixture which is concentrated with respect to mono- and polyunsaturated C18-trans-fatty acids which are protected against biohydrogenation, preferably in the form of their salt, which are insoluble under neutral conditions, such as their calcium salt, said fatty acid mixture also exhibiting a favourable trans-isomer distribution.

According to an advantageous embodiment of the invention at least the unsaturated, but especially all fatty acids of the fatty acid mixture are protected, for example in the above mentioned manner, especially in the form of a calcium salt.

According to the invention the fatty acid mixture is used in an amount of approximately 2-8 % by weight of the dry matter of the feed, or approximately 2-5% by weight of the dry matter of the feed, when calculated on the basis of a compound, *i.e.* a complete feed, which typically constitutes the total dietary source for the animal besides ruffage, such as hay or silage. At the same time the total fat content (that is the sum of the fat and fatty acid contents) is maintained low, being generally at the most 10 % by weight calculated from the dry matter of the feed, or preferably at the most approximately 8%, *e.g.* at the most 7-8% by weight, such as for a compound feed, or calculated on the basis of a compound feed. The low total fat content is reached by choosing for the feed mixture low-fat, preferably grain-based raw materials. If the diet is based on the combined use of a concentrate feed and grain, rather than on a compound feed, a concentrate feed being normally used in lesser amounts, for example in an amount of at the most half as that compared to a compound feed, the amount of fatty acid mixture to be included in the concentrate feed can be increased correspondingly so that the animal is fed a fatty acid mixture in an amount corresponding to the said 2-5 % by weight calculated from the total diet or included in a compound feed. Typically approximately 5-8 % by weight of the said fatty acid mixture can then be used in a concentrate feed, calculated from the dry matter of the concentrate, the total amount of fat being suitably at the most 10 % by weight. Depending on the type of feed, the aim is to design the feed so that the daily ration to be given to the animal is approximately 0.5 - 1.5 g/kg body weight, which for cows mean approximately 200-500 g fatty acid mixture per day.

The fatty acid mixture to be used in the feed according to the invention contains usually at the most approximately 80 % by weight C18-trans-fatty acids, and of these preferably 5-20 % by weight are polyunsaturated C18-trans-fatty acids and 40-60 % by weight are monounsaturated C18-trans-fatty acids. According to an advantageous embodiment the fatty acid mixture to be used according to the invention contains 5-20 % by weight of C18:1 trans-11-fatty acid (trans-vaccenic acid), 5-20 % by weight of C18:1 trans-10-fatty acid, and/or 5-20 % by weight of C18:2 trans-fatty acids. The fatty acid mixture contains preferably the following fatty acids in the stated amounts expressed as % by weight:

| | |
|---|---|
| C18:1 cis | 20-35 |
| C18:1 trans 6-9 | 15-30 |
| C18:1 trans 10 | 5-20 |
| C18:1 trans 11 | 5-20 |
| C18:1 trans | 5-20 |

The total content of saturated fatty acids in such a mixture is as low as possible, but good results have been obtained even when such are present in a total amount of 15-25, more typically 15-20 % by weight, of which the C12-16 fatty acids usually form the major part, such as approximately 2/3, and the C18:0 fatty acids form almost the rest. Typical amounts are approximately 10-12 % by weight of C12:0-16:0 fatty acids and 5-6 % by weight of C18:0 fatty acids. In addition, the mixture can contain small amounts, usually less than approximately 10-12 % by weight of other fatty acids, such as C18:1 trans 12.

According to an advantageous embodiment the fatty acid mixture can contain the following fatty acids (in percentage by weight):

| | |
|---|---|
| C12-16 | 10-12 |
| C18:0 | 5-6 |
| C18:1 cis | 24-30 |
| C18:1 trans 6-9 | 18-22 |
| C18:1 trans 10 | 10-11 |
| C18:1 trans 11 | 7-10 |
| C18:1 trans 12 | 6-8 |
| C18:2 trans | 8-12 |
| other | 1-3 |

A typical compound feed according to the invention preferably contains the following components in % by weight of the dry matter:

| | |
|---|---|
| soybean meal | 0-20 |
| rapeseed meal | 0-25 |
| barley | 0-30 |
| oat | 0-30 |
| sugarbeet pulp | 0-20 |
| wheat bran | 0-30 |
| molasses | 0-8 |
| wheat middlings | 0-20 |
| minerals | 0-5 |
| vitamin and trace element-premix | 0-2 |
| fatty acid mixture | 2-5. |

A typical concentrate feed according to the invention can contain for example the following components in % by weight of the dry matter of the feed:

| | |
|---|---|
| soybean meal | 0-50 |
| rapeseed meal | 0-70 |
| molasses | 0-7 |
| wheat bran | 0-20 |
| sugarbeet pulp | 0-20 |
| minerals | 0-4 |
| vitamin and trace element-premix | 0-2 |
| fatty acid mixture | 5-8.. |

An object of the invention is also a method for the manufacture of the above defined feed, according to which to conventional feed components and conventional feed adjuvants and additives, a fatty acid mixture is added, which contains over 50 % by weight of mono- and polyunsaturated C18-trans-fatty acids which are protected against biohydrogenation. The amount to be added is preferably 2-8 % by weight of the dry matter of the feed, as described earlier.

An object of the invention is also a novel fatty acid mixture having a high proportion of C18-trans-fatty acids protected against biohydrogenation, namely a fatty acid mixture, which contains a total amount of over 50 % by weight and preferably at the most 80 % by weight of mono- and polyunsaturated C18-trans-fatty acids which are protected preferably as their calcium salts. Of these the monounsaturated C18-trans fatty acids constitute the major part, 40-60% by weight, and the polyunsaturated C18-trans fatty acids constitute 5-20% by weight.

An object of the invention is also a method for the preparation of a fatty acid mixture according to which
- a fatty acid mixture which contains 50-70 % by weight of polyunsaturated fatty acids, is hydrogenated to form a fatty acid mixture, which contains over 50 % by weight of mono- and polyunsaturated C18-trans-fatty acids,
- if desired, the obtained hydrogenated fatty acid mixture is neutralised with a base, for example an alkalimetal hydroxide,
- the fatty acids are precipitated as a salt, such as a calcium salt,
- the precipitated fatty acid salts are separated, and optionally dried.

The hydrogenation is preferably carried out with hydrogen in the presence of a catalyst at a pressure of approximately 2-3 bar and a temperature of 150-200 °C. Raney-nickel for example can be used as the catalyst. The neutralisation of the hydrogenated fatty acid mixture suitably takes place with sodium hydroxide. The insoluble salt, such as the calcium salt, is preferably made by precipitating using an aqueous solution of the salt, such as of calcium chloride, at a temperature of 50-60 °C, by adding at least an equimolar amount of calcium salt.

By preparing the protected fatty acid mixture in the afore mentioned manner 70-90 % more trans-fatty acids are obtained in the mixture than before, at the same raw fat level. This in turn makes positive changes in milk fat composition possible with a small fat addition without adverse effects.

The fatty acid mixture to be used as the starting material which contains 50-70 % by weight of polyunsaturated fatty acids, is advantageously obtained from an oil having a suitable fatty acid composition, such as a soybean, sunflower or corn oil, in a manner known per se by splitting the fatty acids from the oil by saponification, for example by using a sodium hydroxide solution, separating the obtained fatty acids and the glycerol by acidifying for example with sulfuric acid and recovering the mixture of free fatty acids, for example as a melt on the surface of the reaction solution (cf. Chemical Process Industries, R. Shreve, J. Brink, McGraw Hill 1997).

A further object of the invention is a method for changing the composition of the fatty acids contained in milk, especially increasing the oleic acid, the CLA and trans-vaccenic acid content thereof, whereby to a milk producing animal, an above defined fatty acid mixture according to the invention is given in an amount sufficient to change the milk fatty acid composition, especially in the form of a feed additive, such as in the form of the afore mentioned feed. In order to achieve a change in said composition, the fatty acid mixture as defined should comprise approximately 2-5 % by weight of the animal's daily diet ration. As stated above, this means an amount of approximately 0.5-1.5 g/kg body weight, or for a normal sized cow, approximately 200-500 g fatty acid mixture per day. The effect on the milk composition can be seen relatively rapidly; in the test described below, a feeding period of three weeks has been used.

The following examples illustrate the invention. In these examples as well as before percentages mean % by weight, unless otherwise mentioned.

### Example 1 - Preparation of a fatty acid mixture

A fatty acid mixture containing 54.5 % by weight polyunsaturated fatty acids, 27.7 % by weight monounsaturated fatty acids and 17.8 % by weight of saturated fatty acids, and in which there was a total of 5.3 % by weight of mono- and polyunsaturated trans-acids, was chosen as the starting material. This fatty acid mixture had been prepared from soybean oil by saponification with 10 % sodium hydroxide at approximately 60 °C. The fatty acids obtained after saponification by acidification and recovering were hydrogenated at a pressure of 3 bar and a temperature of 200 °C in the presence of 0.5% Raney-nickel catalyst (SP-10, Engelhardt). The fatty acid mixture was stirred at a speed of 1000 rpm for approximately 5 hours, whereafter there remained polyunsaturated fatty acids only about 10 % by weight. The end point of hydrogenation was determined gas chromatographically.

After hydrogenation the fatty acids were neutralised to sodium salts using NaOH to a pH value of 7. In order to convert the fatty acids to their calcium salts the sodium hydroxide aqueous solution was treated with a 20 % calcium chloride aqueous solution at a temperature of 50-60 °C using an approximately 1.1-fold equimolar amount of calcium salt. Thereby the fatty acid and the calcium salt formed a precipitate, which was separated from the solution. After separation the fatty acid calcium salt was dried and used as such for feed manufacture.

The composition of the fatty acid mixture was determined gas chromatographically, and it was the following

| | |
|---|---|
| C12-16 | 11.6 |
| C18:0 | 5.7 |
| C18:1 cis | 24.8 |
| C18:1 trans 6-9 | 20.8 |
| C18:1 trans 10 | 10.5 |
| C18:1 trans 11 | 7.9 |
| C18:1 trans 12 | 6.7 |
| C18:2 trans | 9.2 |
| other | 2.5. |

The total trans-fatty acid content in the fatty acid mixture obtained was 55.4 % by weight (of these monounsaturated 46.0 % by weight and polyunsaturated 9.4 % by weight), the total content of polyunsaturated fatty acids was 10.1 % by weight, the total content of monounsaturated fatty acids was 70.8 % by weight, and the content of saturated fatty acids was 19.1 % by weight.

### Example 2 - Method for the manufacture of a feed mixture

The following raw materials were selected for the manufacture of a feed, in the said amounts expressed as % by weight

| | |
|---|---|
| soybean meal | 4 |
| rapeseed meal | 16 |
| barley | 25 |
| oat | 5 |
| sugarbeet pulp | 10 |
| wheat bran | 16 |
| molasses | 6 |
| wheat middlings | 8 |
| minerals | 5 |
| premixes | 1 |
| protected fatty acid mixture | 4 |

The used protected fatty acid mixture was according to Example 1. The feed was made in the conventional manner by mixing the components together. The total fat content of the feed (fat and fatty acids together) was 7 % by weight of the dry matter.

### Example 3 - Feeding test

In the feeding test according to the Example 3, cows were given 10 kg a day of a compound feed mixture according to the invention for a period of three weeks, the composition of which was according to the Example 2. The test feed was compared to a normal compound feed mixture having a corresponding composition, which did not contain the fatty acid addition according to the invention.

In the following table the milk production obtained in the feeding test is indicated, as well as the fat and protein contents of the milk.

| | Comparison | Test feed |
|---|---|---|
| milk kg/day | 31.7 | 33.1 |
| fat % | 3.98 | 3.33 |
| protein % | 3.32 | 3.34 |

From the table it can be seen that the milk production increased with the feed according to the invention. On the other hand the fat content of the milk dereased, and the protein content remained somewhat the same. In the following table the fatty acid profile of the milk is given. For comparison a typical fatty acid composition is taken (percentages are % by weight).

| | Typical fatty acid composition | Obtained with the test feed |
|---|---|---|
| C12-16 | 50 | 33 |
| C18:1 (oleic acid) | 20 | 35 |
| CLA | 0.5 | 1.2 |
| C18:1 trans 11 | 1 | 2.5 |

From the results it can be seen that the fatty acid composition can be changed in a more healthy direction by the test feed according to the invention, that is the proportion of saturated middle length hard fats decreases, whereas the contents of monounsaturated fatty acids, CLA and trans-vaccenic acid (C18:1 trans 11) have increased near the target.

## Claims

1. Feed, which contains conventional feed components and conventional additives and other adjuvants, **characterized in that** the feed contains a mixture of fatty acids, wherein the proportion of mono- and polyunsaturated C18-trans-fatty acids which are protected against biohydrogenation, is over 50 % by weight.

2. The feed according to claim 1, **characterized in that** it contains the fatty acid mixture in an amount of 2-8 % by weight from the dry matter.

3. The feed according to claim 1 or 2, **characterized in that** the feed is grain-based and its total fat and fatty acid content is at the most 10%, preferably at the most 8% by weight, calculated from the dry matter of the feed.

4. The feed according to any one of the preceding claims, **characterized in that** at least the unsaturated fatty acids of the fatty acid mixture, preferably all fatty acids, are protected, especially in the form of their insoluble salts, such as their calcium salts.

5. The feed according to any one of the preceding claims, **characterized in that** the proportion of mono- and polyunsaturated C18-trans-fatty acids is at the most 80 % by weight.

6. The feed according to any one of the preceding claims, **characterized in that** the fatty acid mixture contains
| | |
|---|---|
| polyunsaturated C18-trans-fatty acids | 5-20 % by weight |
| monounsaturated C18-trans-fatty acids | 40-60 -"- |

7. The feed according to any one of the preceding claims, **characterized in that** the fatty acid mixture contains at least one of the following fatty acids in % by weight:
| | |
|---|---|
| C18:1 trans 11 | 5-20 |
| C18:1 trans 10 | 5-20 |
| C18:2 trans | 5-20. |

8. The feed according to any one of the preceding claims, **characterized in that** the fatty acid mixture contains the following fatty acids as % by weight:
| | |
|---|---|
| C18:1 | 20-35 |
| C18:1 trans 6-9 | 15-30 |
| C18:1 trans 10 | 5-20 |
| C18:1 trans 11 | 5-20 |
| C18:2 trans | 5-20. |

9. The feed according to any one of the preceding claims, **characterized in that** is contains in percentages by weight
| | |
|---|---|
| soybean meal | 0-20 |
| rapeseed meal | 0-25 |
| barley | 0-30 |
| oat | 0-30 |
| sugarbeet pulp | 0-20 |
| wheat bran | 0-30 |
| molasses | 0-8 |
| wheat middlings | 0-20 |
| minerals | 0-5 |
| vitamin and trace element-premix | 0-2 |
| fatty acid mixture | 2-5. |

10. Feed according to any one of the claims 1 to 8, **characterized in that** it contains the following components in % by weight of the dry matter of the feed:
| | |
|---|---|
| soybean meal | 0-50 |
| rapeseed meal | 0-70 |
| molasses | 0-7 |
| wheat bran | 0-20 |
| sugarbeet pulp | 0-20 |
| minerals | 0-4 |
| vitamin and trace element premix | 0-2 |
| fatty acid mixture | 5-8. |

11. Method for the preparation af the feed according to claim 1, **characterized in that** to conventional feed components and to conventional feed adjuvants and additives a fatty acid mixture is added which contains over 50 % by weight of C18-trans-fatty acids protected against biohydrogenation, preferably in an amount of 2-8 % by weight based on the dry matter thereof.

12. Fatty acid mixture, **characterized in that** it contains over 50 % and at the most 80% by weight of mono- and polyunsaturated C18-trans-fatty acids protected against biohydrogenation, especially in their calcium salt form, whereby it contains
| | |
|---|---|
| polyunsaturated C18-trans-fatty acids | 5-20 % by weight |
| monounsaturated C18-trans-fatty acids | 40-60 -"-. |

13. The mixture according to claim 12, **characterized in that** it contains at least one of the following fatty acids in % by weight:
| | |
|---|---|
| C18:1 trans 11 | 5-20 |
| C18:1 trans 10 | 5-20 |
| C18:2 trans | 5-20. |

14. The mixture according to claim 12 or 13, **characterized in that** the fatty acid mixture contains the following fatty acids as % by weight:
| | |
|---|---|
| C18:1 | 20-35 |
| C18:1 trans 6-9 | 15-30 |
| C18:1 trans 10 | 5-20 |
| C18:1 trans 11 | 5-20 |
| C18:2 trans | 5-20. |

15. Process for the preparation of a fatty acid mixture protected against biohydrogenation, **characterized in that** it comprises the following steps
- a fatty acid mixture which contains 50-70 % by weight of polyunsaturated fatty acids, is hydrogenated to form a fatty acid mixture, which contains over 50 % by weight of mono- and polyunsaturated C18-trans-fatty acids,
- if desired, the obtained hydrogenated fatty acid mixture is neutralised with a base, for example an alkalimetal hydroxide,
- the fatty acids are precipitated as a salt, preferably as a calcium salt,
- the precipitated fatty acid salts are separated, and optionally dried.

16. The method according to claim 15, **characterized in that** the fatty acid mixture is prepared by saponification of soybean, sunflower or corn oil.

17. The method according to claim 15 or 16, **characterized in that** the hydrogenation is carried out with hydrogen at a pressure of 2-3 bar and a temperature of 150-200°C in the presence of a catalyst.

18. The method according to any one of claims 15-17, **characterized in that** the salts of the hydrogenated fatty acids are precipitated by treating the hydrogenated fatty acid mixture with a salt solution, especially a calcium chloride solution, at a temperature of 50-60 °C.

19. A method for changing the composition of the fatty acids in milk, especially for increasing the oleic acid, CLA and trans-vaccenic acid content thereof, **characterized in that** to a milk producing animal, a mixture of fatty acids according to any one of the claims 12-14 is given in an amount sufficient to change the composition of the milk, preferably in the form of a feed according to any one of the claims 1-10.

20. The method according to claim 19, **characterized in that** an amount of 0.5 - 1.5 g/kg body weight per day is given to the animal.

## Patentansprüche

1. Futter, enthaltend herkömmliche Futterbestandteile und herkömmliche Zusatzstoffe und andere Hilfsstoffe, **dadurch gekennzeichnet, dass** das Futter ein Gemisch aus Fettsäuren enthält, wobei der Anteil von einfach und mehrfach ungesättigten C18-trans-Fettsäuren, die gegen Biohydrierung geschützt sind, mehr als 50 Gew.-% beträgt.

2. Futter nach Anspruch 1, **dadurch gekennzeichnet, dass** es das Fettsäuregemisch in einer Menge von 2 bis 8 Gew.-% der Trockenmasse enthält.

3. Futter nach Anspruch 1 oder 2, **dadurch gekennzeichnet, dass** das Futter eine Getreidegrundlage hat und dass sein gesamter Fett- und Fettsäureanteil höchstens 10% beträgt, vorzugsweise höchstens 8 Gew.-%, berechnet aus der Trockenmasse des Futters.

4. Futter nach einem der vorhergehenden Ansprüche, **dadurch gekennzeichnet, dass** mindestens die ungesättigten Fettsäuren des Fettsäuregemisches, vorzugsweise alle Fettsäuren, geschützt sind, besonders in Form der unlöslichen Salze, wie ihre Calciumsalze.

5. Futter nach einem der vorhergehenden Ansprüche, **dadurch gekennzeichnet, dass** der Anteil von einfach und mehrfach ungesättigten C18-trans-Fettsäuren höchstens 80 Gew.-% beträgt.

6. Futter nach einem der vorhergehenden Ansprüche, **dadurch gekennzeichnet, dass** das Fettsäuregemisch
| | |
|---|---|
| mehrfach ungesättigte C18-trans-Fettsäuren | 5-20 Gew.-% |
| einfach ungesättigte C18-trans-Fettsäuren | 40-60 -"- |
enthält.

7. Futter nach einem der vorhergehenden Ansprüche, **dadurch gekennzeichnet, dass** das Fettsäuregemisch mindestens eine der folgenden Fettsäuren in Gew.-% enthält:
| | |
|---|---|
| C18:1 trans 11 | 5-20 |
| C18:1 trans 10 | 5-20 |
| C18:2 trans | 5-20. |

8. Futter nach einem der vorhergehenden Ansprüche, **dadurch gekennzeichnet, dass** das Fettsäuregemisch die folgenden Fettsäuren in Gew.-% enthält:
| | |
|---|---|
| C18:1 | 20-35 |
| C18:1 trans 6-9 | 15-30 |
| C18:1 trans 10 | 5-20 |
| C18:1 trans 11 | 5-20 |
| C18:2 trans | 5-20. |

9. Futter nach einem der vorhergehenden Ansprüche, **dadurch gekennzeichnet, dass** es die folgenden Bestandteile in Gew.-% enthält
| | |
|---|---|
| Sojabohnenmehl | 0-20 |
| Rapssamenmehl | 0-25 |
| Gerste | 0-30 |
| Hafer | 0-30 |
| Zuckerrübenpulpe | 0-20 |
| Weizenkleie | 0-30 |
| Melasse | 0-8 |
| Weizenfuttermehle | 0-20 |
| Mineralien | 0-5 |
| Vormischung aus Vitaminen und Spuren-elementen | 0-2 |
| Fettsäuregemisch | 2-5. |

10. Futter nach einem der Ansprüche 1 bis 8, **dadurch gekennzeichnet, dass** es die folgenden Bestandteile in Gew.-% der Trockenmasse des Futters enthält:
| | |
|---|---|
| Sojabohnenmehl | 0-50 |
| Rapssamenmehl | 0-70 |
| Melasse | 0-7 |
| Weizenkleie | 0-20 |
| Zuckerrübenpulpe | 0-20 |
| Mineralien | 0-4 |
| Vormischung aus Vitaminen und Spuren-elementen | 0-2 |
| Fettsäuregemisch | 5-8. |

11. Verfahren zur Herstellung des Futters nach Anspruch 1, **dadurch gekennzeichnet, dass** zu den herkömmlichen Futterbestandteilen und den herkömmlichen Futterhilfsstoffen und -zusätzen ein Fettsäuregemisch hinzugefügt wird, das über 50 Gew.-% gegen Biohydrierung geschützte C18-trans-Fettsäuren enthält, vorzugsweise in einer Menge von 2-8 Gew.-% basierend auf seiner Trockenmasse.

12. Fettsäuregemisch, **dadurch gekennzeichnet, dass** es über 50 Gew.-% und höchstens 80 Gew.-% einfach und mehrfach ungesättigte, gegen Biohydrierung geschützte C18-trans-Fettsäuren enthält, besonders in ihrer Calciumsalzform, wobei es enthält
| | |
|---|---|
| mehrfach ungesättigte C18-trans-Fettsäuren | 5-20 Gew.-% |
| einfach ungesättigte C18-trans-Fettsäuren | 40-60 -"-. |

13. Gemisch nach Anspruch 12, **dadurch gekennzeichnet, dass** es mindestens eine der folgenden Fettsäuren in Gew.-% enthält:
| | |
|---|---|
| C18:1 trans 11 | 5-20 |
| C18:1 trans 10 | 5-20 |
| C18:2 trans | 5-20. |

14. Gemisch nach Anspruch 12 oder 13, **dadurch gekennzeichnet, dass** das Fettsäuregemisch die folgenden Fettsäuren in Gew.-% enthält:
| | |
|---|---|
| C18:1 | 20-35 |
| C18:1 trans 6-9 | 15-30 |
| C18:1 trans 10 | 5-20 |
| C18:1 trans 11 | 5-20 |
| C18:2 trans | 5-20. |

15. Verfahren zur Herstellung eines gegen Biohydrierung geschützten Fettsäuregemisches, **dadurch gekennzeichnet, dass** es die folgenden Schritte umfasst
- ein 50-70 Gew.-% mehrfach ungesättigte Fettsäuren enthaltendes Fettsäuregemisch wird hydriert, um ein Fettsäuregemisch zu erzeugen, das mehr als 50 Gew.-% einfach und mehrfach ungesättigte C18-trans-Fettsäuren enthält,
- wenn erwünscht, wird das erhaltene hydrierte Fettsäuregemisch mit einer Base, z.B. einem Alkalimetallhydroxid, neutralisiert,
- die Fettsäuren werden als ein Salz, vorzugsweise als ein Calciumsalz präzipitiert,
- die präzipitierten Fettsäuresalze werden getrennt und gegebenenfalls getrocknet.

16. Verfahren nach Anspruch 15, **dadurch gekennzeichnet, dass** das Fettsäuregemisch durch Verseifung von Sojabohnen-, Sonnenblumen- oder Maisöl hergestellt wird.

17. Verfahren nach Anspruch 15 oder 16, **dadurch gekennzeichnet, dass** die Hydrierung mit Wasserstoff bei einem Druck von 2-3 bar und einer Temperatur von 150-200°C in Gegenwart eines Katalysators durchgeführt wird.

18. Verfahren nach einem der Ansprüche 15 bis 17, **dadurch gekennzeichnet, dass** die Salze der hydrierten Fettsäuren durch die Behandlung des hydrierten Fettsäuregemisches mit einer Salzlösung, besonders einer Calciumchloridlösung, bei einer Temperatur von 50-60 °C präzipitiert werden.

19. Verfahren zur Veränderung der Zusammensetzung der Fettsäuren in Milch, besonders um ihren Gehalt an Ölsäure, CLA und trans-Vaccensäure zu erhöhen, **dadurch gekennzeichnet, dass** einem milcherzeugenden Tier ein Fettsäuregemisch nach einem der Ansprüche 12 bis 14 in einer Menge verabreicht wird, die ausreicht, um die Zusammensetzung der Milch zu verändern, vorzugsweise in Form eines Futters nach einem der Ansprüche 1 bis 10.

20. Verfahren nach Anspruch 19, **dadurch gekennzeichnet, dass** dem Tier eine Menge von 0,5 bis 1,5 g/kg Körpergewicht pro Tag verabreicht wird.

## Revendications

1. Alimentation, qui comprend des composants alimentaires traditionnels et des additifs conventionnels et d'autres adjuvants, **caractérisée en ce que** l'alimentation se compose d'un mélange d'acides gras, dans laquelle la proportion d'acides gras trans C18 mono- et polyinsaturés qui sont protégés contre la biohydrogénation, est supérieure à 50 % en poids.

2. Alimentation selon la revendication 1, **caractérisée en ce qu'**elle se compose du mélange d'acides gras dans une quantité de 2 à 8 % en poids de la matière sèche.

3. Alimentation selon la revendication 1 ou 2, **caractérisée en ce que** l'alimentation est à base de céréales et sa teneur totale en matière grasse et en acides gras est tout au plus de 10 %, de préférence tout au plus de 8 % en poids, calculée à partir de la matière sèche de l'alimentation.

4. Alimentation selon l'une quelconque des revendications précédentes, **caractérisée en ce qu'**au moins les acides gras insaturés du mélange d'acides gras, de préférence tous les acides gras, sont protégés, en particulier sous la forme de leurs sels insolubles, tels que leurs sels de calcium.

5. Alimentation selon l'une quelconque des revendications précédentes, **caractérisée en ce que** la proportion d'acides gras trans C18 mono- et polyinsaturés est tout au plus de 80 % en poids.

6. Alimentation selon l'une quelconque des revendications précédentes, **caractérisée en ce que** le mélange d'acides gras se compose :
| | |
|---|---|
| d'acides gras trans C18 polyinsaturés | 5 à 20 % en poids |
| d'acides gras trans C18 monoinsaturés | 40 à 60 % en poids |

7. Alimentation selon l'une quelconque des revendications précédentes, **caractérisée en ce que** le mélange d'acides gras se compose d'au moins un des acides gras suivants en % en poids :
| | |
|---|---|
| 11 trans C18:1 | 5 à 20 |
| 10 trans C18:1 | 5 à 20 |
| trans C18:2 | 5 à 20. |

8. Alimentation selon l'une quelconque des revendications précédentes, **caractérisée en ce que** le mélange d'acides gras se compose des acides gras suivants en % en poids :
| | |
|---|---|
| C18:1 | 20 à 35 |
| 6 à 9 trans C18:1 | 15 à 30 |
| 10 trans C18:1 | 5 à 20 |
| 11 trans C18:1 | 5 à 20 |
| trans C18:2 | 5 à 20. |

9. Alimentation selon l'une quelconque des revendications précédentes, **caractérisée en ce qu'**elle se compose en pourcentages en poids des éléments suivants :
| | |
|---|---|
| farine de soja | 0 à 20 |
| farine de colza | 0 à 25 |
| orge | 0 à 30 |
| avoine | 0 à 30 |
| pulpe de betterave | 0 à 20 |
| son de blé | 0 à 30 |
| mélasse | 0 à 8 |
| remoulage | 0 à 20 |
| minéraux | 0 à 5 |
| pré-mélange de vitamines et d'oligo-éléments | 0 à 2 |
| mélange d'acides gras | 2 à 5. |

10. Alimentation selon l'une quelconque des revendications 1 à 8, **caractérisée en ce qu'**elle comprend les composants suivants en % en poids de la matière sèche de l'alimentation :
| | |
|---|---|
| farine de soja | 0 à 50 |
| farine de colza | 0 à 70 |
| mélasse | 0 à 7 |
| son de blé | 0 à 20 |
| pulpe de betterave | 0 à 20 |
| minéraux | 0 à 4 |
| pré-mélange de vitamines et d'oligo-éléments | 0 à 2 |
| mélange d'acides gras | 5 à 8. |

11. Procédé pour la préparation de l'alimentation selon la revendication 1, **caractérisé en ce qu'**on ajoute aux composants d'alimentation conventionnelle et aux adjuvants et additifs d'alimentation conventionnelle un mélange d'acides gras qui se compose de plus de 50 % en poids d'acides gras trans C18 protégés contre la biohydrogénation, de préférence dans une quantité de 2 à 8 % en poids basée sur sa matière sèche.

12. Mélange d'acides gras, **caractérisé en ce qu'**il se compose de plus de 50 % et tout au plus de 80 % en poids d'acides gras trans C18 mono- et polyinsaturés protégés contre la biohydrogénation, en particulier sous la forme de leur sel de calcium, moyennant quoi ils se composent :
| | |
|---|---|
| d'acides gras trans C18 polyinsaturés | 5 à 20 % en poids |
| d'acides gras trans C18 monoinsaturés | 40 à 60 % en poids. |

13. Mélange selon la revendication 12, **caractérisé en ce qu'**il se compose d'au moins un des acides gras suivants en % en poids :
| | |
|---|---|
| 11 trans C18:1 | 5 à 20 |
| 10 trans C18:1 | 5 à 20 |
| trans C18:2 | 5 à 20. |

14. Mélange selon la revendication 12 ou 13, **caractérisé en ce que** le mélange d'acides gras se compose des acides gras suivants en % en poids :
| | |
|---|---|
| C18:1 | 20 à 35 |
| 6 à 9 trans C18:1 | 15 à 30 |
| 10 trans C18:1 | 5 à 20 |
| 11 trans C18:1 | 5 à 20 |
| trans C18:2 | 5 à 20. |

15. Processus pour la préparation d'un mélange d'acides gras protégé contre la biohydrogénation, **caractérisé en ce qu'**il comprend les étapes suivantes :
- un mélange d'acides gras qui contient 50 à 70 % en poids d'acides gras polyinsaturés, est hydrogéné pour former un mélange d'acides gras, qui contient plus de 50 % en poids d'acides gras trans C18 mono- et polyinsaturés,
- si cela est souhaité, le mélange d'acides gras hydrogéné obtenu est neutralisé par une base, par exemple par un hydroxyde de métal alcalin,
- les acides gras sont précipités sous forme d'un sel, de préférence sous forme d'un sel de calcium,
- les sels d'acides gras précipités sont séparés, et facultativement séchés.

16. Procédé selon la revendication 15, **caractérisé en ce que** le mélange d'acides gras est préparé par saponification de farine de soja, de tournesol ou d'huile de maïs.

17. Procédé selon la revendication 15 ou 16, **caractérisé en ce que** l'hydrogénation est réalisée avec de l'hydrogène à une pression de 2 à 3 bar et à une température de 150 à 200°C en présence d'un catalyseur.

18. Procédé selon l'une quelconque des revendications 15 à 17, **caractérisé en ce que** les sels des acides gras hydrogénés sont précipités en traitant le mélange d'acides gras hydrogéné avec une solution saline, en particulier une solution de chlorure de calcium, à une température de 50 à 60°C.

19. Procédé destiné à modifier la composition des acides gras présents dans le lait, en particulier destiné à augmenter sa teneur en acide oléique, en ALC (acide linoléique conjugué) et en acide trans-vaccénique, **caractérisé en ce que** l'on donne un mélange d'acides gras à un animal laitier selon l'une quelconque des revendications 12 à 14 dans une quantité suffisante pour modifier la composition du lait, de préférence sous forme d'une alimentation selon l'une quelconque des revendications 1 à 10.

20. Procédé selon la revendication 19, **caractérisé en ce qu'**une quantité de 0,5 à 1,5 g/kg de poids corporel est donnée par jour à l'animal.
